# EUROPEAN PATENT SPECIFICATION

(11) **EP 2 284 822 B1**
(45) Date of publication and mention of the grant of the patent: **08.03.2017**
(21) Application number: 10172555.4
(22) Date of filing: 11.08.2010
(51) Int. Cl.: G09B 23/30, A61M 15/00

(54) **Systems and Methods for Evaluting Medication Delivery from a Delivery Apparatus Using a Model Face and Airway**
Systeme und Verfahren zur Bewertung der Medikamentenverabreichung aus einer Zufuhrvorrichtung mit einer Modellseite und einem Luftweg
Systèmes et procédés pour évaluer l'administration de médicaments à partir d'un appareil d'administration utilisant un modèle de visage et voie d'aération

(30) Priority: 11.08.2009 US 233014 P
(43) Date of publication of application: 16.02.2011
(73) Proprietor: Trudell Medical International, London, Ontario N5V 5G4 (CA)
(72) Inventor: Nuttall, Michael, London, Ontario N6A 3X2 (CA)
(74) Representative: Grünecker Patent- und Rechtsanwälte PartG mbB

(56) References cited:
- EP-A1- 1 619 644
- WO-A1-2008/025339
- DE-A1- 2 832 201
- US-A- 5 468 151
- US-A1- 2004 214 150
- US-A1- 2007 218 438

## Description

### RELATED APPLICATIONS

The present application claims the benefit of U.S. Provisional Patent Application No. 61/233,014 (still pending), filed Aug. 11, 2009, the entirety of which is hereby incorporated by reference.

The present application is related to U.S. Pat. App. No. 12/326,450, filed December 2, 2008, which claims priority to U.S. Provisional Pat. App. No. 61/005,740, filed December 7, 2007, the entirety of each of which are hereby incorporated by reference.

### BACKGROUND

A variety of delivery apparatus are used to deliver medication in aerosol form to a patient's lungs or nasal passageway, including, but not limited to, pressurized metered dose inhalers, dry powder inhalers, and nebulizers. Delivery apparatus may be used together with a valved holding chamber (VHC) with or without a mask or together with a mask without a VHC. As used herein the term "delivery apparatus" includes any delivery apparatus used to deliver medication in aerosol form to a patient's lungs or nasal passageway alone or together with a VHC (with or without a facemask) or with a facemask without a VHC. The testing of a delivery apparatus is often complicated by the need to achieve a seal between the delivery apparatus and a test apparatus. Small leakages between the delivery apparatus and test apparatus are known to result in large decreases in delivery efficiency of medication. For this reason, some existing standards recommend altering delivery apparatus, such as removing a facemask, during testing. However, a solution for testing delivery apparatus that more closely mimics reality is desirable. The realism is desirable to simulate an accurate implementation of delivering medication from a delivery apparatus to a face and airway of a patient.

Document DE 28 32 201 A1 relates to a model of a human head for medical teaching purposes. The head of the prior art can be assembled and disassembled and is made of a deformable material in order to provide as real training conditions as possible. At the upper jaw portion, two pins made of polyester resin are molded and covered with a skin made of silicon rubber. Also, the jaw joints are covered with the silicon rubber.

Document WO 2008/205339 A1 relates to artifical tissue for surgical operation training, said tissue consisting of a deformable mixture of a hydrogel and natural fibers. The mixture also contains glutaraldehyde.

### BRIEF DESCRIPTION OF THE DRAWINGS

Figure 1 illustrates a front view of a model face and airway;
Figure 2 illustrates a simulated bone structure and a simulated trachea of a model face and airway;
Figure 3 illustrates a partial cross section of a model face and airway;
Figure 4 illustrates another partial cross section of a model face and airway;
Figure 5 illustrates yet another partial cross section of a model face and airway;
Figure 5b is a side view of a simulated trachea;
Figure 6 is a perspective view of a test cradle for use with a model face and airway;
Figure 7 is a perspective view of a model face and airway mounted on a test cradle;
Figure 8 is a side view of a test cradle connected to an impactor; and
Figure 9 is a perspective view of a rear of the test cradle; and
Figure 10 is a flow chart of a method for evaluating medication delivery from a delivery apparatus using a model face and airway.

### DETAILED DESCRIPTION OF THE DRAWINGS

The present disclosure is directed to systems and methods for evaluating medication delivery from a delivery apparatus using a model face and airway. Generally, a model face and airway for use in evaluating medication delivery is created by simulating a bone structure, a trachea, soft tissue, and an epidermal skin layer of an infant, child, or adult. The model face and airway may be mounted on a test cradle that applies a medication delivery apparatus to the model face at an appropriate clinical orientation with a clinically appropriate force for use in evaluating medication delivery from the delivery apparatus. Generally, using a simulated model face and airway provides the ability to evaluate what medications become trapped in an air passageway when received from a delivery apparatus such as a pressurized metered dose inhaler (pMDI) or a delivery apparatus with a valved holding chamber comprising a facemask that holds an aerosol. Additionally, using a model face and airway provides the ability to evaluate what particle sizes emerge from an end of a simulated air passageway to determine the effectiveness of different delivery apparatus, particle sizes of medications and inhalation therapies.

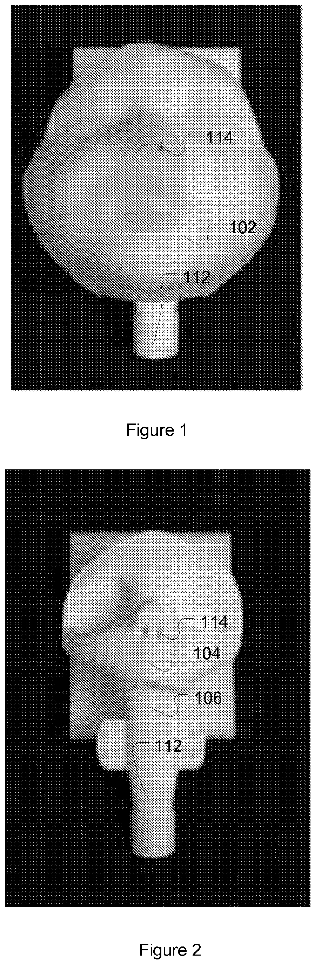

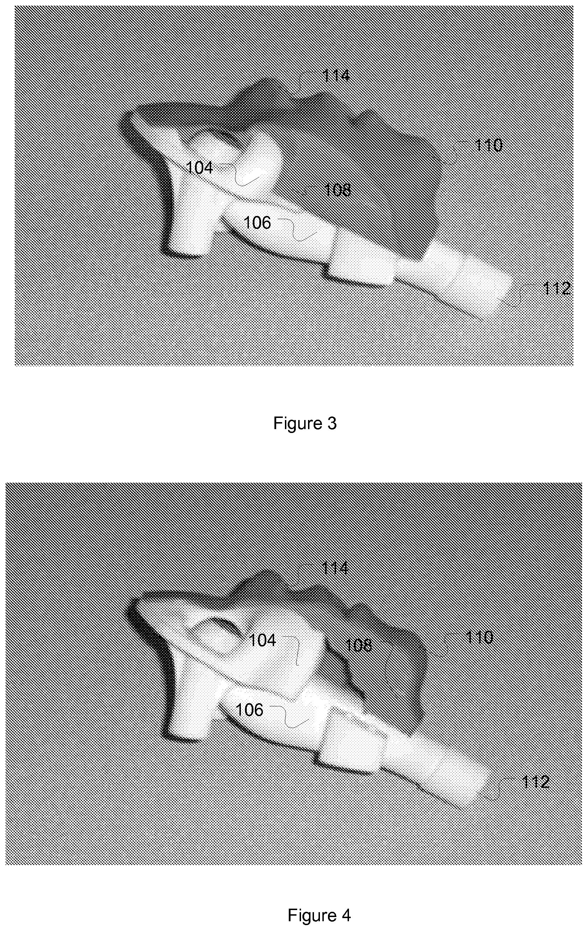

Figure 1 illustrates a front view of a model face and airway of an infant 102; Figure 2 illustrates a simulated bone structure 104 and a simulated trachea 106 of the model face and airway of an infant 102; Figure 3 illustrates a partial cross section of the model face and airway of an infant 102; and Figure 4 illustrates another partial cross section of the model face and airway of an infant 102. Generally, a model of an infant face and airway 102 simulates an infant of ages seven month old. However, it will be appreciated that similar model faces and airways may be used to simulate a child (such as a child between three and four years old) or an adult with different dimensions to properly simulate a face and airway of a child or an adult.

Referring to Figs. 1-5a, the model face and airway 102 may include the simulated bone structure 104, the simulated trachea 106, simulated soft tissue 108, and simulated epidermal skin layer 110. In one implementation, the simulated bone structure 104 and the simulated trachea 106 may comprise nylon to simulate bone and the airway, the simulated soft tissue 108 may comprise foam such as TC-265 A/B produced by BJB Enterprises, Inc. to simulate subcutaneous fat and muscle, and the simulated epidermal skin layer 110 may comprise a chemically-resistant material such as Skinflex III produced by BJB Enterprises Inc. to simulate an epidermal skin layer on the face. A chemically resistant material such as Skinflex III is used to simulate the epidermal skin layer 110 because alcohol or other chemicals that might be used in an aerosol produced by a metered dose inhaler during testing will not affect the material. However, other materials may be used for the simulated bone structure 104, simulated trachea 106, simulated soft tissue 108, and simulated epidermal skin layer 110. In some implementations, the simulated soft tissue 108 and simulated epidermal skin layer 110 have a durometer of between approximately 21 and 31 for durometer type OOO.

In some implementations, when the model face and airway 102 is assembled, the simulated epidermal skin layer 110 surrounds and encompasses at least one side of the simulated soft tissue 108. However, in other implementations, the simulated epidermal skin layer 110 completely surrounds and encompasses the simulated soft tissue 108. The simulated epidermal skin layer 110 and simulated soft tissue 108 is positioned over at least a portion of the simulated bone structure 104 to create a substantially accurate representation of a face and an airway of an infant. The substantially accurate representation of a face and an airway of an infant allows for greater accuracy in testing a seal that is formed with masks of various sizes and shapes or pMDIs that may be associated with holding chambers.

When assembled, the simulated bone structure 104, simulated trachea 106, simulated soft tissue 108, and simulated epidermal skin layer 110 define an air passageway 112 so that air and/or other aerosolized medicaments may flow through the simulated bone structure 104, simulated soft tissue 108, and simulated epidermal skin layer 110 at a simulated nasal cavity 114 and into the simulated trachea 106. In some implementation, the simulated trachea 106 models the air passageway past the throat of a person and to carina region. Further, in some implementations, a glycerin-like material, such as Brij-35 polyoxyethylene lauryl ether surfactant as a solution in methanol ((15% w/v) with glycerol (5 g) added as thickening agent, may be placed in the air passageway 112 to simulate the viscous coating within the trachea and sinus passages of a person. In some implementations, the viscous coating may coat the entire air passageway 112.

As explained in more detail below, one advantage of having a simulated epidermal skin layer 110 and simulated soft tissue 108 that is distinct from the simulated bone structure 104 and the simulated trachea 106 is that during testing, the simulated epidermal skin layer 110 and simulated soft tissue 108 may be removed from the model face and airway 102 so that the air passageway 112 with the simulated trachea 106 may be analyzed without impedance from the material of the simulated epidermal skin layer 110 and simulated soft tissue 108.

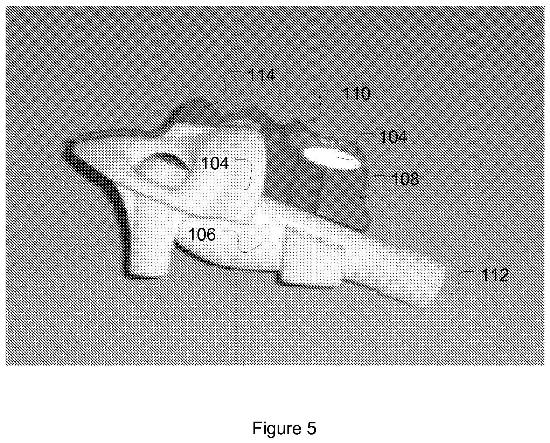

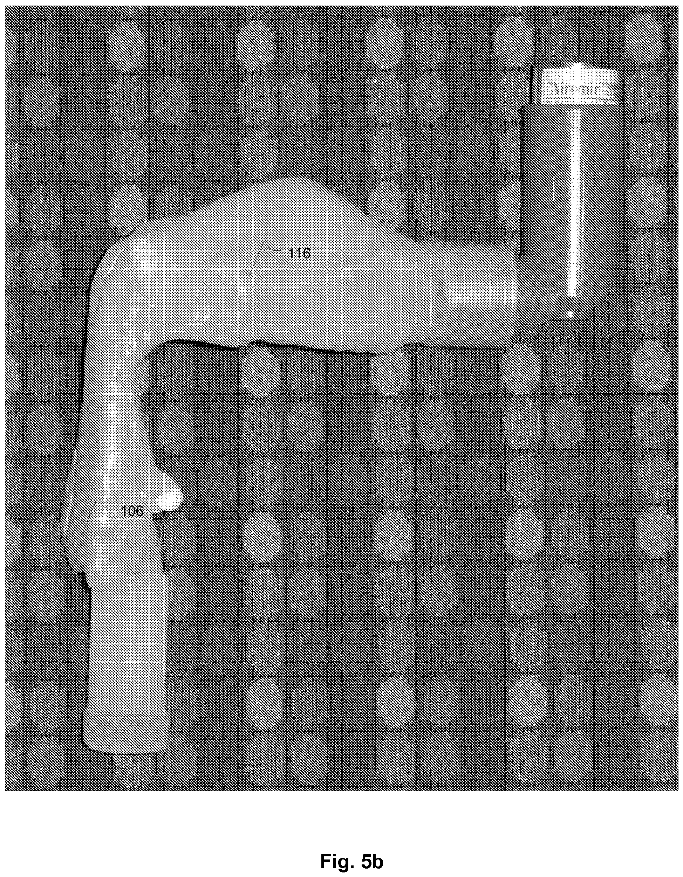

In implementations of a model face of a child and/or an adult, the model face may additionally define a simulated mouth cavity 116 and additional simulated bone 104 as shown in Fig. 5. Alternatively, in other implementations, the model face of a child and/or an adult may define a mouth cavity 116 and additional simulated bone 104 without a nasal cavity. The simulated mouth cavity 116 may be in communication with the simulated trachea 106 so that air or aerosolized medicaments may additionally flow into the air passageway 112 from the mouth cavity 116. The mouth cavity 116 may be used to test delivery apparatus, such as a pMDI, that is placed directly in the mouth cavity 116. Typically, when a pMDI is placed directly in the mouth cavity 116, it is assumed that an essentially perfect seal is formed between the mouth and the pMDI, and the model face and airway 102 may be used to check an amount and partial size of an aerosol that is ejected from the pMDI.

In some implementations, the model face and airway 102 is created based on a computed tomography scan ("CT scan") of a sinus cavity and tracheal area, including a mouth cavity, of an average person or of a specific patient. Generally, a sintered nylon, which is a beaded nylon material, is placed in an array and scanned with a laser pursuant to the CT scan contours. The scanning of the nylon with the laser welds the beads of the nylon together such that after the scanning is complete, any loose beads of nylon may be blown out of the mold.

The remaining fused structure of nylon substantially maps to the CT scan of the sinus cavity and the tracheal area. The remaining fused structure of nylon additionally includes bone structures captured in the CT scan such as the cheekbone and the jawbone behind and around the nasal cavity and the mouth cavity. It will be appreciated that the remaining fused structure of nylon corresponds to the simulated bone structure 104 and simulated trachea 106 described above. One example of a simulated mouth cavity 116 and simulated trachea created in this manner is shown in Fig. 5b.

In some implementations, the simulated bone mouth cavity 116 and simulated trachea 106 may be used to evaluate medication delivery from a delivery apparatus without the simulated soft tissue 108 and simulated epidermal skin layer 110. However, in implementations where the simulated soft tissue 108 and simulated epidermal skin layer 110 are used, the simulated soft tissue 108 and simulated epidermal skin layer 110 may be prepared according to the CT scan so that when assembled with the simulated bone structure 104 and the simulated trachea 106, the model face 102 accurately represents the CT scan.

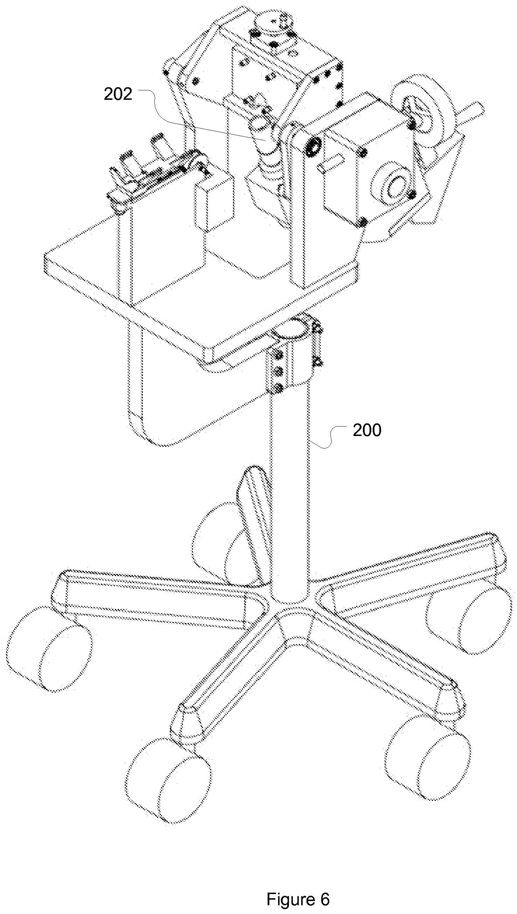

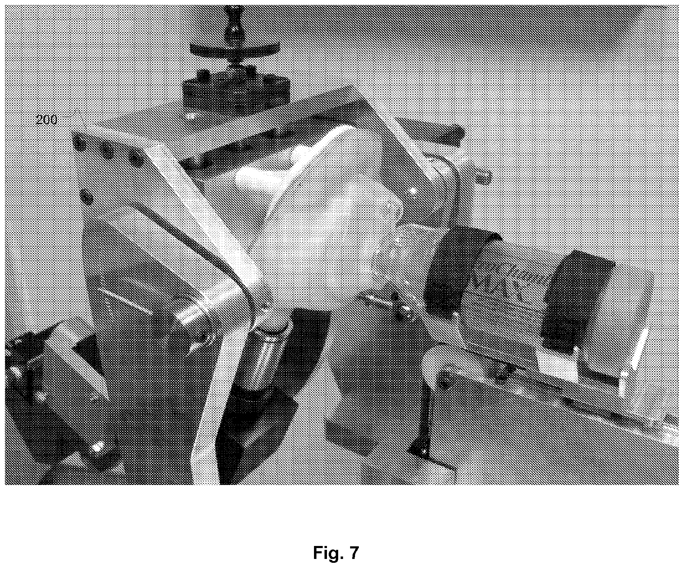

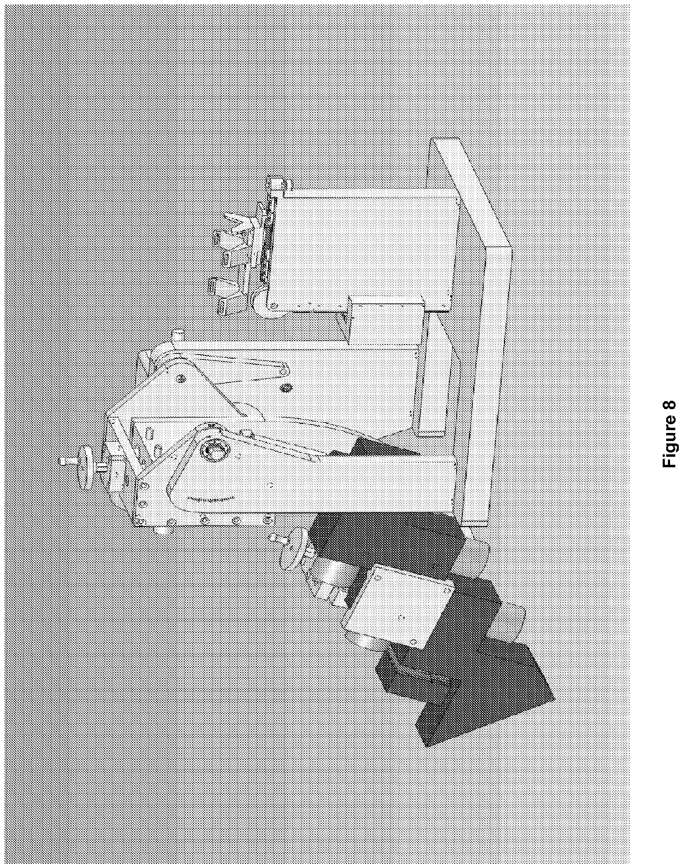

During operation, a model face and airway such as the model faces and airways described above with respect to Figs. 1-5b may be positioned on a test cradle 200, such as the test cradle 200 shown in Figs. 6-9, that applies a medication delivery apparatus with an facemask, or other types of medication delivery apparatus without a facemask, to the model face at a clinically appropriate orientation with an amount of clinically appropriate force. A seal between a facemask, or other types of medication delivery apparatus, and the model face may then be tested by measuring a flow rate at the medication delivery apparatus, such as a flow rate entering a facemask, and a flow rate exiting the air passageway of the model face and airway.

Additionally, delivering aerosolized medication by using a delivery apparatus and measuring an amount of medication received in the air passageway of the model face and airway may evaluate medication delivery from the delivery apparatus comprising a VHC. Alternatively, medication delivery from a delivery apparatus with a VHC may be evaluated by measuring aerodynamic particle size distribution of medication by transferring an aerosol from a mouth cavity or nasal cavity of the model face and airway, via a USP/Ph.Eur induction port or similar entry point simulating the human upper airway, to a multi-stage cascade impactor.

When a model face and airway is mounted on the test cradle 200, an induction port 202 couples with, and forms a substantially airtight seal with, the simulated trachea (106, Figs. 1-5b) of the model face and airway described above so that gasses may pass through the air passageway of the model face and airway and into the induction port 202. The induction port 202 may be coupled with various types of testing apparatus for use in simulating the breathing of a patient at different tidal breathing patterns. For example, when a vacuum is created at the induction port 202, gases or other particulates are drawn into the nasal cavity and/or mouth cavity of the model face and airway to simulate a patient inhaling. One example of a testing apparatus that may perform these types of operations is a model ASL 5000 test lung by IngMar Medical.

It should be appreciated that the induction port 202 may be a USP/Ph.Eur induction port or equivalent representation of the human oropharynx. The artificial attempt to model the human upper airway (oropharynx) is used when the model face and airway is connected to a multi-stage cascade impactor for measurements of aerodynamic particle sized distribution (APSD) of an inhaled aerosol. The induction port 202 may also be a flexible pipe connection from the model face and airway to a breathing simulator for measurements of emitted total mass of medication, mimicking use by a tidal-breathing patient.

When a model face and airway is mounted to the testing cradle 200 and a delivery apparatus mounted to the testing cradle 200 engages, and forms a seal with the model face, it will be appreciated that an airflow path is created so that gasses may flow from the delivery apparatus, into the air passageway of the model face and airway via the nasal cavity and/or mouth cavity, and into the induction port 202 of the testing cradle. A flow meter or a pressure measurement device may also be coupled with the induction port to measure gases flowing through the model face and airway.

In some implementations of the testing cradle 200, the testing cradle may be configured such that the model face and airway, or a medication delivery apparatus applied to the model face and airway, may be adjusted vertically, horizontally, and/or angularly, thereby adjusting the position of the model face and airway with respect to the delivery apparatus when the delivery apparatus engages the model face. These adjustments may be made to test both ideal and non-ideal seals between the delivery apparatus and the model face.

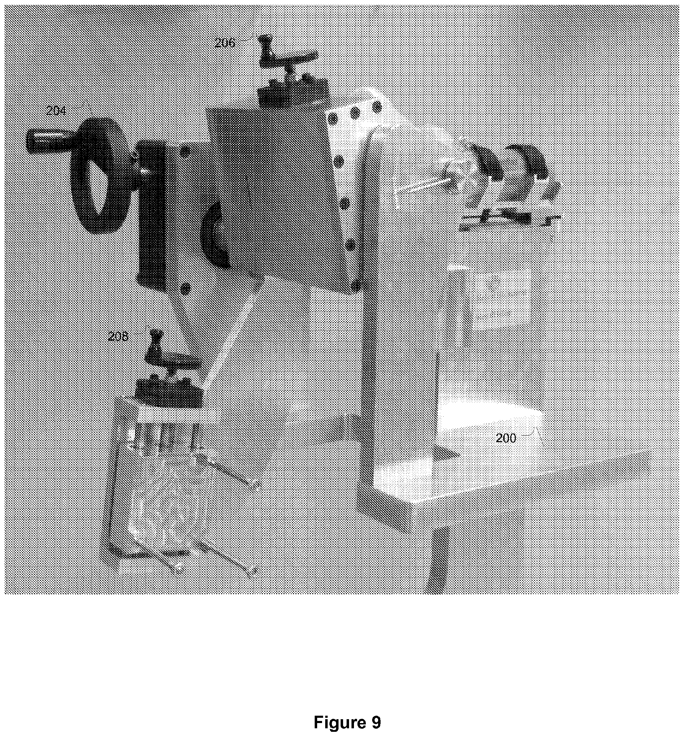

For example, as shown in Fig. 9, the testing cradle 200 may include a crank system 204 that adjusts an angle of the model face and airway forwards and backwards through an axis that is located approximately through the ears of the model face, the testing cradle 200 may include a crank system 206 that adjusts the vertical height of the model face and airway with respect to the facemask, and the testing cradle 200 may include a crank system 208 that adjusts an impactor inlet up and down to provide for a proper connection with the simulated trachea of the model face and airway.

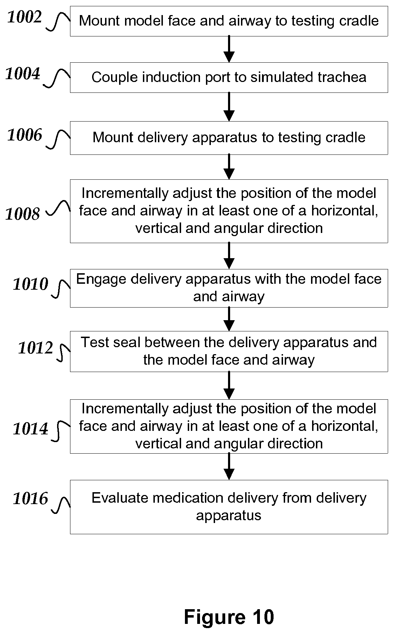

Figure 10 is a flow chart of a method for evaluating the medication delivery from a delivery apparatus with a facemask and a model face and airway. Generally, the method 1000 begins at step 1002 with a model face and airway being mounted to a test cradle. At step 1004, an induction port is coupled with a simulated trachea of the model face and airway.

At step 1006, the facemask and delivery apparatus are mounted on the test cradle, and at step 1008, the horizontal, vertical, and/or angular position of the model face and airway with respect to the facemask may be incrementally adjusted so that the facemask properly engages the model face when the facemask is positioned on the model face and airway. Alternatively, the horizontal, vertical, and/or angular position of the model face and airway with respect to the facemask may be incrementally adjusted to test non-ideal engagements between the facemask and the model face and airway. At step 1010, a weight of the test cradle is engaged to create a clinically appropriate force between the facemask and the model face and airway.

At step 1012, a seal between the facemask and the model face and airway is tested. In one implementation, the seal between the facemask and the model face and airway is tested by creating a vacuum to simulate an inhalation at the trachea of the model face and airway. The flow rate of gases or particulates entering the facemask and leaving the mouth opening is then measured, and based on the difference in flow rates, a quality of the seal can be determined. It will be appreciated that the closer a difference in a flow rate of gases or particulates entering the facemask and a flow rate of gases or particulates leaving the simulated trachea is to zero, the better the seal between the facemask and the model face and airway. At step 1014, the horizontal, vertical, and/or angular position of the model face and airway may be adjusted based on the determined quality of the seal between the facemask and the model face and airway.

At step 1016, the medication delivery from the delivery apparatus is evaluated. In one implementation, the medication delivery from the delivery apparatus is evaluated by injecting an amount of aerosolized medicine into the facemask, and measuring the total amount of aerosolized medicine received at the simulated trachea of the model face and airway. In another implementation, the medication delivery from the delivery apparatus is evaluated as before, but with the aerosol transferred from the lips of the model face to a multi-stage cascade impactor by a vacuum source attached to the impactor for the determination of aerosynamic particle size distribution.

Figs. 1-10 teach systems and methods for evaluating medication delivery from a delivery apparatus using a model face and airway. The disclosed model faces and airways simulate a face and airway of an infant, child, or adult by simulating a bone structure, a trachea, soft tissue, and an epidermal skin layer of a person. The model face and airway may be mounted on a test cradle that applies a delivery apparatus to the model face at an appropriate clinical orientation with a clinically appropriate force for use in evaluating medication delivery from the delivery apparatus.

It is intended that the foregoing detailed description be regarded as illustrative rather than limiting, and that it be understood that it is the following claims, including all equivalents, that are intended to define the scope of this invention.

## Claims

1. A model face and airway (102) for use in evaluation of medication delivery by a delivery apparatus, the model face and airway (102) comprising:
a simulated bone structure (104) defining a nasal cavity;
simulated soft tissue (108) positioned over at least a portion of the simulated bone structure (104); and
a simulated epidermal skin layer (110) surrounding at least one side of the simulated soft tissue (108),
wherein the simulated bone structure (104), the simulated soft tissue (108), and the simulated epidermal skin layer (110) define an air passageway (112) in communication with the nasal cavity,
a simulated trachea defining an air passageway.
wherein the air passageway defined by the simulated trachea is in communication with the nasal cavity.

2. The model face of claim 1, wherein the simulated bone structure, simulated soft tissue, and simulated epidermal skin layer define a mouth cavity.

3. The model face of claim 1, wherein the simulated soft tissue and the simulated epidermal skin layer are configured to allow air to enter into the air passageway at the nasal cavity of the simulated bone structure.

4. The model face of any of claims 1 to 3, wherein the simulated bone structure, simulated soft tissue, and simulated epidermal skin layer define a mouth cavity in communication with the air passageway of the simulated trachea.

5. The model face of any of the preceding claims, wherein the simulated bone structure and/or the simulated trachea comprise nylon.

6. The model face of any of the preceding claims, wherein the simulated soft tissue comprises foam.

7. The model face of any of the preceding claims, wherein the simulated epidermal skin layer comprises a chemical-resistant material.

8. The model face of any of the preceding claims, wherein the simulated epidermal skin layer comprises Skinflex III.

9. The model face of any of the preceding claims, wherein the model face and airway are dimensioned to simulate a face and airway of an infant.

10. The model face of any of the preceding claims, wherein the model face and airway are dimensioned to simulate a face and airway of a child.

11. The model face of any of the preceding claims, wherein the model face and airway are dimensioned to simulate a face and airway of an adult.

12. The model face of any of the preceding claims, wherein the nasal cavity of the simulated bone structure and/or the air passageway for the simulated trachea comprise a viscous coating.

13. The model face of any of the preceding claims, wherein the simulated soft tissue and the simulated epidermal skin layer have a durometer of between approximately 21 and 31 for durometer type OOO.

14. A method for evaluating a medication delivery from a delivery apparatus, the method comprising the steps of:
mounting the model face and airway (102) according to claim 1 on a testing cradle (200);
mounting a medication delivery apparatus on the testing cradle;
initiating a force on the testing cradle to simulate a clinically appropriate force between the medication delivery apparatus and the model face and airway;
incrementally adjusting a position of the model face with the testing cradle in at least one of a horizontal, vertical, or angular direction; and
testing a medication delivery of the medication delivery apparatus based on an amount of an aerosolized medication deployed from the medication delivery apparatus and an amount of the aerosolized medication received at a simulated trachea of the model face and airway.

## Patentansprüche

1. Gesichts- und Atemwegmodell (102) zur Verwendung bei der Analyse einer Medikamentenabgabe durch eine Abgabevorrichtung, wobei das Gesichts- und Atemwegmodell (102) umfasst:
eine simulierte Knochenstruktur (104), die eine Nasenhöhle definiert;
ein simuliertes Weichgewebe (108), das wenigstens über einem Teil der simulierten Knochenstruktur (104) angeordnet ist; und
eine simulierte Oberhautschicht (110), die wenigstens eine Seite des simulierten Weichgewebes (108) umgibt,
wobei die simulierte Knochenstruktur (104), das simulierte Weichgewebe (108) und die simulierte Oberhautschicht (110) einen Luftdurchgang (112) definieren, der mit der Nasenhöhle in Verbindung steht,
eine simulierte Luftröhre, die einen Luftdurchgang definiert,
wobei der Luftdurchgang, der durch die simulierte Luftröhre definiert ist, mit der Nasenhöhle in Verbindung steht

2. Gesichtsmodell nach Anspruch 1, wobei die simulierte Knochenstruktur, das simulierte Weichgewebe und die simulierte Oberhautschicht eine Mundhöhle definieren.

3. Gesichtsmodell nach Anspruch 1, wobei das simulierte Weichgewebe und die simulierte Oberhautschicht so ausgebildet sind, dass Luft in den Luftdurchgang an der Nasenhöhle der simulierten Knochenstruktur eindringen kann.

4. Gesichtsmodell nach einem der Ansprüche 1 bis 3, wobei die simulierte Knochenstruktur, das simulierte Weichgewebe und die simulierte Oberhautschicht eine Mundhöhle definieren, der mit dem Luftdurchgang der simulierten Luftröhre in Verbindung steht.

5. Gesichtsmodell nach einem der vorhergehenden Ansprüche, wobei die simulierte Knochenstruktur und/oder die simulierte Luftröhre aus Nylon gebildet sind.

6. Gesichtsmodell nach einem der vorhergehenden Ansprüche, wobei das simulierte Weichgewebe Schaum umfasst.

7. Gesichtsmodell nach einem der vorhergehenden Ansprüche, wobei die simulierte Oberhautschicht ein chemikalienbeständiges Material umfasst.

8. Gesichtsmodell nach einem der vorhergehenden Ansprüche, wobei die simulierte Oberhautschicht Skinflex III umfasst.

9. Gesichtsmodell nach einem der vorhergehenden Ansprüche, wobei das Gesichts- und Atemwegmodell so dimensioniert ist, dass es ein Gesicht und Atemwege eines Säuglings simuliert.

10. Gesichtsmodell nach einem der vorhergehenden Ansprüche, wobei das Gesichts- und Atemwegmodell so dimensioniert ist, dass es ein Gesicht und Atemwege eines Kindes simuliert.

11. Gesichtsmodell nach einem der vorhergehenden Ansprüche, wobei das Gesichts- und Atemwegmodell so dimensioniert ist, dass es ein Gesicht und Atemwege eines Erwachsenen simuliert.

12. Gesichtsmodell nach einem der vorhergehenden Ansprüche, wobei die Nasenhöhle der simulierten Knochenstruktur und/oder der Luftdurchgang für die simulierte Luftröhre eine viskose Beschichtung umfassen.

13. Gesichtsmodell nach einem der vorhergehenden Ansprüche, wobei das simulierte Weichgewebe und die simulierte Oberhautschicht ein Durometer zwischen etwa 21 und 31 für einen OOO-Durometer aufweisen.

14. Verfahren zur Analyse einer Medikamentenabgabe aus einer Abgabevorrichtung, wobei das Verfahren die Schritte umfasst:
Montieren des Gesichts- und Atemwegmodells (102) gemäß Anspruch 1 auf einer Testaufnahmevorrichtung (200);
Anbringen einer Medikamentenabgabevorrichtung auf der Testaufnahmevorrichtung;
Aufbringen einer Kraft auf die Testaufnahmevorrichtung, um eine klinisch geeignete Kraft zwischen der Medikamentenabgabevorrichtung und dem Gesichts- und Atemwegmodell zu simulieren;
Schrittweises Einstellen einer Position des Gesichtsmodells auf die Testaufnahmevorrichtung in einer horizontalen und/oder vertikalen und/ oder schrägen Richtung; und
Testen einer Medikamentenabgabe der Medikamentenabgabevorrichtung auf der Grundlage einer Menge eines aerosolisierten Medikaments, die aus der Medikamentenabgabevorrichtung abgegeben wird, und einer Menge des aerosolisierten Medikaments, die an einer simulierten Luftröhre des Gesichts- und Atemwegmodells empfangen wird.

## Revendications

1. Modèle de visage et de voies respiratoires (102) destiné à être utilisé lors de l'évaluation de l'administration de médicaments par un appareil de délivrance, le modèle de visage et de voies respiratoires (102) comprenant :
une structure osseuse simulée (104) définissant une cavité nasale,
du tissu mou simulé (108) positionné par-dessus d'au moins une partie de la structure osseuse simulée (104), et
une couche épidermique simulée (110) entourant au moins un côté du tissu mou simulé (108),
dans lequel la structure osseuse simulée (104), le tissu mou simulé (108) et la couche épidermique simulée (110) définissent une voie de passage d'air (112) communiquant avec la cavité nasale,
une trachée simulée définissant une voie de passage d'air,
dans lequel la voie de passage d'air, définie par la trachée simulée, est en communication avec la cavité nasale.

2. Modèle de visage selon la revendication 1, dans lequel la structure osseuse simulée, le tissu mou simulé et la couche épidermique simulée définissent une cavité buccale.

3. Modèle de visage selon la revendication 1 dans lequel le tissu mou simulé et la couche épidermique simulée sont configurés pour permettre à l'air d'entrer dans la voie de passage d'air au niveau de la cavité nasale de la structure osseuse simulée.

4. Modèle de visage selon l'une quelconque des revendications 1 à 3, dans lequel la structure osseuse simulée, le tissu mou simulé et la couche épidermique simulée définissent une cavité buccale en communication avec la voie de passage d'air de la trachée simulée.

5. Modèle de visage selon l'une quelconque des revendications précédentes, dans lequel la structure osseuse simulée et/ou la trachée simulée comprennent du nylon.

6. Modèle de visage selon l'une quelconque des revendications précédentes, dans lequel le tissu mou simulé comprend de la mousse.

7. Modèle de visage selon l'une quelconque des revendications précédentes, dans lequel la couche épidermique simulée comprend un matériau résistant aux produits chimiques.

8. Modèle de visage selon l'une quelconque des revendications précédentes, dans lequel la couche épidermique simulée comprend du Skinflex III.

9. Modèle de visage selon l'une quelconque des revendications précédentes, le modèle de visage et de voies respiratoires étant dimensionné pour simuler le visage et les voies respiratoires d'un nourrisson.

10. Modèle de visage selon l'une quelconque des revendications précédentes, le modèle de visage et de voies respiratoires étant dimensionné pour simuler le visage et les voies respiratoires d'un enfant.

11. Modèle de visage selon l'une quelconque des revendications précédentes, le modèle de visage et de voies respiratoires étant dimensionné pour simuler le visage et les voies respiratoires d'un adulte.

12. Modèle de visage selon l'une quelconque des revendications précédentes, dans lequel la cavité nasale de la structure osseuse simulée et/ou la voie de passage d'air pour la trachée simulée comprennent un revêtement visqueux.

13. Modèle de visage selon l'une quelconque des revendications précédentes, dans lequel le tissu mou simulé et la couche épidermique simulée présentent une durométrie comprise entre approximativement 21 et 31 pour un duromètre de type OOO.

14. Procédé d'évaluation de l'administration de médicaments par un appareil de délivrance, le procédé comprenant les étapes suivantes :
le montage du modèle de visage et de voies respiratoires (102) conforme à la revendication 1 sur un berceau de test (200),
le montage d'un appareil de délivrance de médicaments sur le berceau de test,
l'initiation d'une force sur le berceau de test pour simuler une force cliniquement appropriée entre l'appareil de délivrance de médicaments et le modèle de visage et de voies respiratoires,
l'ajustement par incréments de la position du modèle de visage avec le berceau de test dans au moins l'une d'une direction horizontale, verticale ou angulaire, et
le test de l'administration de médicaments, par l'appareil de délivrance de médicaments, fondé sur une quantité de médicaments en aérosol envoyée à partir de l'appareil de délivrance de médicaments, et sur une quantité de médicaments en aérosol reçue au niveau d'une trachée simulée du modèle de visage et de voies respiratoires.
